# EUROPÄISCHE PATENTSCHRIFT

(11) **EP 1 161 297 B1**
(45) Veröffentlichungstag und Bekanntmachung des Hinweises auf die Patenterteilung: **01.10.2003**
(21) Anmeldenummer: 00916880.8
(22) Anmeldetag: 01.03.2000
(51) Int. Cl.: B01J 23/755, B01J 37/03, C07C 209/36

(54) **NICKEL-ENTHALTENDER HYDRIERKATALYSATOR UND VERFAHREN ZU SEINER HERSTELLUNG**
HYDROGENATING CATALYST CONTAINING NICKEL AND METHOD FOR PRODUCING THE SAME
CATALYSEUR D'HYDROGENATION CONTENANT DU NICKEL ET SON PROCEDE DE PRODUCTION

(30) Priorität: 03.03.1999 DE 19909176
(43) Veröffentlichungstag der Anmeldung: 12.12.2001
(73) Patentinhaber: KataLeuna GmbH Catalysts, 06236 Leuna (DE)
(72) Erfinder: BIRKE, Peter, D-06179 Langenbogen (DE); GEYER, Reinhard, D-06120 Halle (DE); KRAAK, Peter, D-04159 Leipzig (DE); SCHÖDEL, Rainer, D-06179 Teutschenthal (DE)
(74) Vertreter: Schrell, Andreas, Dr.
(86) Internationale Anmeldenummer: EP0001709
(87) Internationale Veröffentlichungsnummer: WO00051728

(56) Entgegenhaltungen:
- EP-A- 0 335 222
- EP-A- 0 672 452
- US-A- 4 090 980
- US-A- 4 597 908
- US-A- 4 670 416
- US-A- 5 047 178

## Beschreibung

Die Erfindung betrifft einen Katalysator, der insbesondere für die Hydrierung von Nitrogruppen in Nitroaromaten zu den entsprechenden Aminen in Gegenwart von Wasser eingesetzt werden kann, und ein Verfahren zu dessen Herstellung.

Die katalytische Hydrierung von Nitroaromaten ist bekannt. Die Hydrierungen werden im allgemeinen sowohl im Festbettreaktor als auch im Batch-Reaktor durchgeführt. In technischem Maßstab werden am häufigsten Hydrierungen in der Flüssigphase mit suspendiertem Katalysator vorgenommen, wobei die Verfahren sich durch die Reaktionstemperatur, den Druck, den Katalysator, die Lösungsmittel und die Art der Reaktionsführung unterscheiden. Als Katalysatoren finden dabei verschiedene Katalysatorsysteme Anwendung, wie beispielsweise Nickel-haltige Katalysatoren. So beschreibt zum Beispiel die JP 551 33 33 die Hydrierung von 2,4-Dinitrotoluol und 2,6-Dinitrotoluol in Gegenwart der Katalysatoren Pd/C, Raney-Nickel, Raney-Kobalt beziehungsweise Platinschwarz.

Die EP-A 98 681 offenbart einen Nickel-Kieselgur-Trägerkatalysator für die Hydrierung von Di-Nitrobenzophenon zum entsprechenden Diamin.

In der DE-A 3 537 247 wird die Hydrierung von Di-Nitroverbindungen zu den Diaminen in Gegenwart von modifizierten Raney-Nickel-Katalysatoren beschrieben.

In der DD 152 065 wird der Einsatz eines Nickel-SiO₂-Katalysators mit spezieller Partikelgrößenverteilung für die Hydrierung von Nitroverbindungen offenbart.

Die EP-A 0 335 222 offenbart die Anwendung von Nickel-Al₂O₃/ZrO₂-Trägerkatalysatoren für die Hydrierung von Nitrilen, Aromaten, Nitroverbindungen und Olefinen. Die Schrift offenbart unter anderem die gleichzeitige Fällung von Nickel, Zirconium und Aluminium auf Trägern bei 50 bis 120°C und bei einem pH-Wert von 7,3 bis 9,0, wobei als Träger Aktivkohle, Al₂O₃, SiO₂, Kieselgur und andere eingesetzt werden.

Die SU-PS 28 31 85 offenbart Nickel-Al₂O₃/ZrO₂-Katalysatoren, die durch Fällen von Nickel und Al₂O₃ auf ZrO₂ hergestellt wurden.

Gemäß der Lehre der US-PS 2,564,331 wird ein Nickel-ZrO₂-Katalysator durch Fällen eines Nickelund Zirconylcarbonatgemisches mit anschließendem Waschen, Trocknen und Reduzieren bei 250 bis 350°C hergestellt, wobei der Katalysator maximal 10 Masse-% ZrO₂ aufweist.

Auch in der DE-AS 1 257 753 wird die Fällung unlöslicher Carbcnate offenbart, wobei der Fällvorgang durch Verdampfen von CO₂ und NH₃ aus einer Mischsalzlösung von Ammoniumzirconylcarbonat und Nickelammincarbonat ausgelöst wird.

Die EP-A 0 672 452 offenbart Katalysatoren zur Hydrierung organischer Verbindungen, die im wesentlichen 65 bis 80 Masse-% Nickel, berechnet als NiO, 10 bis 25 Masse-% SiO₂, 2 bis 10 Masse-% Zirconium, berechnet als ZrO₂ und 0 bis 10 Masse-% Aluminium, berechnet als Al₂O₃, enthalten, wobei die Summe aus dem Gehalt an SiO₂ und Al₂O₃ mindestens 15 Masse-% beträgt. Die Herstellung dieser Katalysatoren erfolgt durch Zugabe einer sauren wäßrigen Lösung von Ni-, Zr- und gewünschtenfalls Aluminiumverbindungen zu einer basischen wäßrigen Lösung oder Suspension von Siliciumverbindungen und gewünschtenfalls Aluminiumverbindungen. Während der Fällung wird der pH-Wert zunächst auf 4,0 bis 6,5 abgesenkt und nachfolgend auf 7 bis 8 eingestellt. Das Fällprodukt wird getrocknet, calciniert und verformt.

Die bisher bekannten Nickel-Hydrierkatalysatoren weisen alle den Nachteil auf, daß unter den hydrothermalen Reaktionsbedingungen der Nitroaromatenhydrierung eine schnelle Alterung der Katalysatoren auftritt.

Das der vorliegenden Erfindung zugrunde liegende technische Problem liegt also darin, Nickel-haltige Trägerkatalysatoren bereitzustellen, die insbesondere unter den hydrothermalen Reaktionsbedingungen der Nitroaromatenhydrierung eine höhere Lebensdauer als die herkömmlichen Katalysatoren aufweisen.

Dieses Problem wird erfindungsgemäß dadurch gelöst, daß ein Katalysator, insbesondere für die Hydrierung von Nitrogruppen in Nitroaromaten zu den entsprechenden Aminen in Gegenwart von Wasser, enthaltend Nickel auf einem Träger bereitgestellt wird, wobei der Katalysator reduziert und stabilisiert ist, Mickelkristallite mit bimodaler Nickelkristallitgrößenverteilung, einen Nickel-Gehalt von 60, insbesondere 61 Masse-%, bis 80 Masse-% (bezogen auf die Gesamtmasse des Katalysators) einen ZrO₂ - Gehalt von 20 bis 40 Masse -% (bezogen auf die Gesamtmasse, des Katalytors), sowie einen Reduktionsgrad von mindestens 70 % aufweist. Der Reduktionsgrad wird nach einer einstündigen Nachreduktion des stabilisierten Katalysators bei 100°C bestimmt.

Die Erfindung sieht in einer besonders bevorzugten Ausführungsform vor, daß der vorgenannte Katalysator eine bimodale Nickelkristallitgrößenverteilung aufweist, wobei die beiden Maxima der Nickelkristallitgrößenverteilung bei 30 bis 80 Angström und 81 bis 150 Angström liegen. Die Erfindung sieht in einer weiteren besonders bevorzugten Ausführungsform vor, daß der Anteil des Nickels mit einem Maximum der Nickelkristallitgrößenverteilung bei 30 bis 80 Angström ≥ 40 % (bezogen auf die Gesamtmasse des Katalysators) beträgt. Der Anteil an metallischem Nickel mit Kristalliten einer Größe von 30 bis 80 Angström beträgt also ≥ 40 % (bezogen auf die Gesamtmasse des Katalysators).

In einer weiteren bevorzugten Ausführungsform ist vorgesehen, daß der vorgenannte Katalysator auf einem zirconiumhaltigen Träger geträgert ist, vorzugsweise ZrO₂, ZrO₂HfO₂, SiO₂ · ZrO₂, SiO₂ · ZrO₂HfO₂ oder Gemische mindestens zweier Substanzen davon enthält oder aus diesen besteht.

In einer besonders bevorzugten Ausführungsform beträgt der SiO₂-Gehalt 0 bis 20 Masse-% (bezogen auf die Gesamtmasse des Katalysators). In einer weiteren bevorzugten Ausführungsform beträgt der HfO₂-Gehalt 0 bis 4 Masse-% (bezogen auf die Gesamtmasse des Katalysators).

In einer besonders bevorzugten Ausführungsform der Erfindung können die reduzierten und stabilisierten Katalysatoren als Pulver mit Korngrößen von 1 bis 100 µm, vorzugsweise von 2 bis 25 µm, eingesetzt werden. Selbstverständlich können auch Formlinge eingesetzt werden.

Die erfindungsgemäßen Katalysatoren zeichnen sich in vorteilhafter und überraschender Weise durch ihre gegenüber herkömmlichen Katalysatoren verlängerte Lebensdauer bei gleicher oder verbesserter katalytischer Aktivität aus. Katalysatoren der erfindungsgemäßen bimodalen Nickelkristallitgrößenverteilung weisen insbesondere unter hydrothermalen Reaktionsbedingungen eine erheblich verlängerte Lebensdauer gegenüber herkömmlichen Katalysatoren auf.

Im Zusammenhang mit der vorliegenden Erfindung wird unter einer bimodalen Nickelkristallitgrößenverteilung eine Verteilung der Nickelkristallite verstanden, gemäß der zwei deutlich voneinander unterscheidbare Maxima der Kristallitgrößenverteilung vorliegen.

Im Zusammenhang mit der vorliegenden Erfindung wird unter dem Begriff Reduktionsgrad der Anteil des metallischen Nickels am Gesamtnickelgehalt des Katalysators in % nach einstündiger Nachreduktion bei 100°C des stabilisierten Katalysators verstanden.

Die Erfindung betrifft in einer weiteren Ausführungsform auch ein Verfahren zur Herstellung des vorgenannten Katalysators. Die Erfindung betrifft also auch ein Verfahren zur Herstellung eines Nickel-haltigen Trägerkatalysatores, insbesondere eines Katalysators für die Hydrierung von Nitrogruppen in Nitroaromaten zu den entsprechenden Aminen in Gegenwart von Wasser, wobei durch Fällen aus einer Ni²⁺- und Zr⁴⁺-haltigen Lösung mit einer basischen Lösung, insbesondere einer Lösung von NaOH, NaHCO₃ oder Na₂CO₃ oder eines Gemisches mindestens zweier dieser Substanzen, bis zu einem pH-Wert von 8 bis 9 ein Fällprodukt erhalten wird, welches bei Temperaturen von 250°C bis 650°C calciniert, gegebenenfalls anschließend inertisiert, und nachfolgend mit Wasserstoff bei Temperaturen von 250°C bis 550°C, insbesondere 300°C bis 550°C, reduziert, gegebenenfalls inertisiert und anschließend stabilisiert wird.

In einer besonders bevorzugten Ausführungsform enthält die Ni²⁺- und Zr⁴⁺-haltige Lösung zusätzlich Hf⁴⁺. In einer weiteren bevorzugten Ausführungsform enthält die Ni²⁺ und Zr²⁺-haltige Lösung oder die Ni²⁺ und Zr⁴⁺/Hf⁴⁺-haltige Lösung Siliciumdioxid SiO₂, vorzugsweise in suspendierter Form. In bevorzugter Ausführungsform kann vorgesehen sein, daß die Ni²⁺- und Zr⁴⁺-haltige Lösung Nitrate aufweist, insbesondere in Form von Zirconylnitrat.

Die Herstellung des Fällprodukts erfolgt also durch Zugabe der genannten basischen Lösung zu der Ni²⁺und Zr⁴⁺-haltigen Lösung, wobei diese Zugabe soweit und solange erfolgt, bis die Mischung der beiden Lösungen einen pH-Endwert von 8 bis 9 erreicht.

Die Erfindung sieht in einer bevorzugten Ausführungsform vor, daß die Fällung bei Temperaturen von 50°C bis 95°C erfolgt. In bevorzugter Ausführung kann vorgesehen sein, nach der durchgeführten Fällung, also dem Erreichen des pH-Endwertes, die erhaltene Suspension zum Beispiel für 1 bis 2 Stunden nachzurühren, bevor eine Weiterbearbeitung erfolgt.

In einer weiteren Ausbildung betrifft die Erfindung ein vorgenanntes Verfahren, wobei das Fällprodukt nach der Fällung filtriert, gewaschen, vorzugsweise mit Wasser, und nachfolgend bei Temperaturen von 110°C bis 150°C in nicht-reduzierender Atmosphäre getrocknet und ein Vorläuferkatalysator erhalten wird.

Im Zusammenhang mit der vorliegenden Erfindung wird unter einem Vorläuferkatalysator ein Produkt verstanden, das nach der Fällung der Ausgangskomponenten, also der Ni²⁺- und Zr⁴⁺-haltigen, gegebenenfalls Hf⁴⁺-haltigen Lösung, und gegebenenfalls des SiO₂ mit der zugesetzten basischen Lösung, Filtration, Waschen mit Wasser und Trocknung bei Temperaturen in nicht-reduzierender Atmosphäre erhalten wird.

Erfindungsgemäß ergeben sich bei der Herstellung des Vorläuferkatalysators Phasen aus Nickelhydroxynitrat (Ni₃(OH)₄(NO₃)₂) oder Nickelhydroxynitrat (Ni₃(OH)₄(NO₃)₂-haltige Phasen, insbesondere Gemische aus Nickelhydroxynitrat (Ni₃(OH)₄(NO₃)₂), Nickelhydroxycarbonat (Ni₂(OH)₂CO₃ 4H₂O) und Nickelhydroxysilikat (Ni₃Si₂O₅(OH)₄) oder Gemische aus Nickelhydroxynitrat (Ni₃(OH)₄(NO₃)₂) und Nickelhydrogencarbonat (Ni(HCO₃)₂ oder Gemische aus Nickelhydroxynitrat (Ni₃(OH)₄(NO₃)₂) und Nickelhydroxid (Ni(OH)₂) mit Gitteraufweitungen.

Im Zusammenhang mit der vorliegenden Erfindung wird unter Gitteraufweitung eine Interferenzlagenverschiebung zu kleineren Winkeln verstanden.

Entweder vor oder nach der Calcination kann der Katalysatorvorläufer zu Tabletten, Strängen, Kissen, Kugeln oder ähnlichem verformt werden.

Die Reduktion des calcinierten Produktes kann erfindungsgemäß sowohl am Pulver als auch an den Formlingen erfolgen. Erfindungsgemäß ist es besonders bevorzugt, während der Reduktion Gasbelastungen im Bereich von 500 bis 3000 v/v h einzusetzen.

Erfindungsgemäß ist in bevorzugter Ausführung vorgesehen, daß die Katalysatoren nach der Reduktion, vorzugsweise mit einem O₂-N₂-CO₂-Gemisch, stabilisiert werden.

Die Erfindung betrifft daher auch die Bereitstellung eines Verfahrens zur Passivierung eines, vorzugsweise reduzierten und/oder vorzugsweise inertisierten erfindungsgemäßen Katalysators, wobei der Katalysator in einem Verfahrensschritt a) in einem CO₂-N₂-Gasgemisch mit einem CO₂-Gehalt von 0,5 bis 10 Vol.-% bei Temperaturen von 91°C bis 350°C mindestens 30 Minuten behandelt, in einem Verfahrensschritt b) anschließend in dem in Schritt a) genannten Gasgemisch auf eine Temperatur von maximal 90°C abgekühlt, anschließend in einem Verfahrensschritt c) nach Erreichen der Temperatur von maximal 90°C in einer ersten Passivierungsphase dem Gasgemisch Sauerstoff, vorzugsweise Luft, bis zu einem Gehalt an 0,2 bis 1,5 Vol.-% Sauerstoff zugesetzt und der Katalysator in dem Gemisch mindestens 30 Minuten unter Rütteln behandelt und anschließend in einem Verfahrensschritt d) der CO₂-Gehalt in dem Gasgemisch gemäß Schritt c) in einer zweiten Passivierungsphase auf <0,1 Vol.-% reduziert und der O₂-Gehalt auf 1,5 bis 21 Vol.-% erhöht wird.

Die erfindungsgemäße Verfahrensweise zur Stabilisierung des Katalysators weist den Vorteil kurzer Stabilisierungszeiten auf, wobei gleichzeitig gut reaktivierbare Katalysatoren mit sehr guter thermischer Stabilität erhalten werden. In vorteilhafter Weise werden die Katalysatoren besonders gleichmäßig passiviert. Tatsächlich war es überraschend, daß durch das Behandeln mit CO₂-armen Inertgasen unter den angegebenen Bedingungen sehr gleichmäßig und leicht reaktivierbare Katalysatoren erhalten wurden.

Die Erfindung betrifft in einer bevorzugten Ausführungsform ein vorgenanntes Verfahren, wobei zumindest die Passivierung in einem Katalysatorbett kontinuierlich oder im Batch-Verfahren durchgeführt wird, insbesondere mit einem Katalysatorbett, dessen Höhe zum Durchmesser-Verhältnis im Bereich von 0,05 bis 1 liegt.

In einer weiteren bevorzugten Ausführungsform sieht die Erfindung ein vorgenanntes Verfahren vor, wobei die Konzentration des CO₂ während der Behandlung mit dem CO₂-N₂-Gemisch gemäß des Verfahrensschrittes a) 1 bis 2,5 Vol.-% beträgt.

In einer weiteren bevorzugten Ausführungsform sieht die Erfindung ein vorgenanntes Verfahren vor, wobei die Gasbelastung während der Behandlung mit dem CO₂-N₂-Gemisch gemäß des Verfahrensschrittes a) 500 bis 10000 v/v h beträgt. In einer weiteren bevorzugten Ausführungsform sieht die Erfindung vor, daß das vorgenannte Verfahren eine Gasbelastung während der Behandlung mit dem CO₂-N₂-Gemischs gemäß des Verfahrensschritts a) und/oder während der Behandlung mit dem CO₂-N₂-O₂-Gasgemisch gemäß der Verfahrensschritte c) und d) 100 bis 3000 v/v h beträgt.

Die Erfindung sieht in einer weiteren bevorzugten Ausführungsform vor, daß das vorgenannte Verfahren die Behandlung in dem CO₂-N₂-O₂-Gasgemisch gemäß der Verfahrensschritte c) und d) für einen Zeitraum von 30 Minuten bis 8 Stunden durchgeführt wird.

Die Erfindung betrifft in einer weiteren Ausbildung ein vorgenanntes Verfahren, wobei die zeitliche Dauer der Behandlung gemäß des Verfahrensschrittes c), also der ersten Passivierungsphase, zu der zeitlichen Dauer des Verfahrensschrittes gemäß des Verfahrensschrittes d), also der zweiten Passivierungsphase, 9:1 beträgt.

In einer weiteren bevorzugten Ausführungsform betrifft die Erfindung ein vorgenanntes Verfahren, wobei die Temperatur der Behandlung des Katalysators mit dem CO₂-N₂-O₂-Gasgemisch gemäß Schritt c) und/oder Schritt d) 50 bis 70°C beträgt.

In einer weiteren bevorzugten Ausführungsform sieht die Erfindung ein vorgenanntes Verfahren vor, wobei die CO₂-Konzentration im CO₂-N₂-O₂-Gasgemisch während der Behandlung gemäß des Verfahrensschrittes c) 0,5 bis 1,5 Vol.-% beträgt. Die Erfindung kann in bevorzugter Weise vorsehen, den CO₂-Gehalt des Gemisches aus Schritt a) für die Durchführung des Schrittes c) herabzusetzen, zum Beispiel auf den vorgenannten Bereich.

Gemäß einer weiteren bevorzugten Ausbildung der vorliegenden Erfindung wird ein vorgenanntes Verfahren bereitgestellt, wobei die O₂-Konzentration im CO₂-N₂-O₂-Gasgemisch während der Behandlung gemäß des Verfahrensschrittes c) 0,25 bis 0,8 Vol.-% beträgt.

In einer weiteren bevorzugten Ausbildung der Erfindung beträgt die O₂-Konzentration während der Behandlung gemäß des Verfahrensschrittes d) 5 bis 10 Vol.-%.

Die Erfindung betrifft in einer weiteren Ausgestaltung ein vorgenanntes Verfahren, wobei vorgesehen ist, daß das Rütteln des Katalysatorbettes gemäß der Verfahrensschritte c) und/oder d) in Zeitabständen von 10 bis 20 Minuten über einen Zeitraum von jeweils 0,5 bis 2 Minuten vorgenommen wird. Es ist vorteilhaft, Rüttelfrequenzen von 10 bis 50 Hz einzustellen.

Selbstverständlich ist es auch möglich, insbesondere bei pulverförmigen Katalysatoren und Katalysatoren mit sehr hohen Festigkeiten, das Katalysatorbett durch Erzeugen einer Wirbelschicht oder durch Anordnung in einem Drehrohrofen in Bewegung zu setzen. Ein wesentlicher Gesichtspunkt der vorliegenden Erfindung ist es, den Katalysator zumindest zeitweise während der Passivierungsphasen gemäß der Verfahrensschritte c) und d) in dem Sauerstoff-Kohlenstoffdioxid-Stickstoff-Gemisch zu bewegen, beispielsweise in einem Bewegtbett.

Die Stabilisierung kann in einer besonders bevorzugten Weise auch durchgeführt werden, indem in einem Stickstoffstrom mit einem Sauerstoffgehalt von 0,1 bis 1 Vol.-% und einem CO₂-Gehalt von 0,6 Vol.-% bei Temperaturen unterhalb von 80°C stabilisiert wird.

Selbstverständlich ist es möglich, die Stabilisierung des erfindungsgemäß erhaltenen reduzierten Katalysators auch in anderer Weise durchzuführen, beispielsweise gemäß der Lehre der US-PS 4,090,980, die hinsichtlich der Verfahrensparameter zur Stabilisierung von Katalysatoren in den Offenbarungsgehalt der vorliegenden Anmeldung mit einbezogen ist.

Die Erfindung wird anhand der folgenden Beispiele näher erläutert:

### Beispiel 1 (erfindungsgemäß)

4,5 l Wasser werden in einem mit einem Rührer versehenen, beheizbaren Fällbehälter vorgelegt und anschließend eine Metallnitratlösung, die neben 400 g Nickel auch Zirconium in Form einer Zirconylnitratlösung enthält, zugegeben. Das Molverhältnis von Nickel zu ZrO₂ in der Metallnitratlösung ist ca. 6. Nach der Zugabe der Metallnitratlösung wird unter Rühren auf eine Temperatur von 60°C aufgeheizt und anschließend mit einer wäßrigen NaOH-Lösung, die durch Lösen von 600 g Natriumhydroxid in 8 l Wasser hergestellt wurde, bei Temperaturen von 60°C bis zu einem pH-Wert von 8 bis 8,5 gefällt. Die Fällzeit beträgt 2 Stunden. Im Anschluß an die Fällung wird die Suspension noch ca. 2 Stunden bei obigen Temperaturen nachgerührt, danach filtriert und so lange mit alkalifreiem Wasser gewaschen, bis der Na₂O-Gehalt im Filterkuchen <0,3 % bezogen auf den Glührückstand des bei 800°C getemperten Filterkuchens liegt. Anschließend wird der Filterkuchen ca. 15 Stunden bei Temperaturen von 120 bis 150°C getrocknet und bei 350°C calciniert.

Die röntgenographische Phasenanalyse des getrockneten Zwischenproduktes ergab im wesentlichen ein Gemisch aus Nickelhydroxynitrat (Ni₃(OH)₄(NO₃)₂) und Ni(OH)₂.

Nach der Calcinierung wird das getrocknete Zwischenprodukt gemahlen, tablettiert, im Stickstoffstrom (1000 v/v h) inertisiert, im Wasserstoffstrom (1000 v/v h) mit einer Aufheizrate von 5°C/min auf 450°C hochgeheizt und 6h bei 450°C reduziert. Anschließend wurde 30 min bei 450°C im Stickstoffstrom mit 1500 v/v h inertisiert, danach im Stickstoffstrom (1500 v/v h) auf 280°C abgekühlt, bei dieser Temperatur wird dem Stickstoff Kohlendioxid in einer solchen Menge zugesetzt, daß die CO₂-Konzentration 2 Vol-% beträgt. Der Katalysator wird mit diesem Gemisch 30 Minuten bei 280°C behandelt, nachfolgend im gleichen Gasgemisch auf 55°C abgekühlt und in einem Stickstoffstrom (1500 v/v h) mit einem Sauerstoffgehalt von 0,1 bis 1 Vol-% und einem CO₂-Gehalt von 0,6 Vol-% bei Temperaturen unterhalb 80°C stabilisiert. Die Sauerstoffkonzentration wurde so gewählt, daß die Katalysatortemperatur 80°C nicht überstieg. Die Stabilisierungszeit bei Temperaturen < 80°C betrug 4 h. Der Reduktionsgrad des Katalysators nach einer einstündigen Nachreduktion bei 100°C beträgt 84%.

Der reduzierte und stabilisierte Katalysator enthält ca. 60 % Nickel und 30 % ZrO₂ bezogen auf die Masse des Gesamtkatalysators.

Er weist eine bimodale Nickelkristallitgrößenverteilung auf. Die Maxima der Kristallitgrößen liegen bei 52 Angström und 107 Angström. Der Anteil an feindispersem Nickel, das heißt Nickel mit Kristallitgrößen von 30 bis 80 Angström, beträgt ca. 61 %. Die Ergebnisse der katalytischen Messungen sind in der Tabelle zusammengestellt.

### Beispiel 2 (erfindungsgemäß)

8 l Wasser und eine Lösung von Natriumsilikat (60 g/l) werden in den beheizbaren Fällbehälter vorgelegt und anschließend unter Rühren eine vereinigte Nickel-Zirconylnitratlösung (Molverhältnis: Nickel/ZrO₂/SiO₂ = 1:0,13:0,065) zugegeben. Danach wird unter Rühren auf eine Temperatur von 75-80°C aufgeheizt und mit der Fällung unter Zugabe einer wäßrigen Sodalösung (150 g Soda/l Lösung) begonnen. Die Fällung ist beim Erreichen des pH-Wertes von 8-8,5 abgeschlossen. Nach der einstündigen Fällung wird die fertige Fällsuspension noch 2 Stunden nachgerührt und wie im Beispiel 1 beschrieben weiterverarbeitet. Die Calcinierung des getrockneten Materials wurde bei 450°C vorgenommen.

Die röntgenographische Untersuchung des getrockneten Zwischenproduktes ergab ein Gemisch aus Nickelhydroxynitrat (Ni₃(OH)₄(NO₃)₂), Nickelhydroxycarbonat (Ni₂(OH)₂CO₃ 4H₂O) und Nickelhydroxysilikat (Ni₃Si₂O₅(OH)₄).

Die weitere Verarbeitung des Fällproduktes wurde wie im Beispiel 1 vorgenommen. Der fertige Katalysator enthält ca. 65 % Nickel, 18 % ZrO₂ und 3 % SiO₂, bezogen auf den Gesamtkatalysator. Er wies eine bimodale Nickelkristallitgrößenverteilung auf. Die Maxima der Kristallitgrößen lagen bei 49 Angström und 112 Angström. Der Anteil an feindispersem Nickel, das heißt Nickel mit Kristallitgrößen von 30 bis 80 Angström, beträgt ca. 54 %. Der Katalysator weist nach einer einstündigen Nachreduktion im Wasserstoffstrom (Belastung: 1000 v/vh) bei 100°C einen Reduktionsgrad von 75 % auf.

Die Ergebnisse der katalytischen Testung sind in der Tabelle dargestellt.

### Beispiel 3 (erfindungsgemäß)

6 l Wasser werden in den Fällbehälter vorgelegt und anschließend eine Metallnitratlösung, die neben Nickel und Zirconium in Form der Nitrate auch Kieselgur als SiO₂-Träger enthält, zugegeben. Das Molverhältnis von Nickel/ZrO₂/SiO₂ in der Metallnitrat/Träger-Suspension betrug 1:0,14:0,1. Nach dem Erreichen einer Temperatur von 90°C erfolgt die Fällung durch Zugabe einer Natriumbicarbonatlösung, die durch Lösen von 1,35 kg NaHCO₃ in 10 l Wasser hergestellt wurde. Nach Erreichen der Fälltemperatur von 90°C wird mit der Fällung begonnen. Die Fällzeit beträgt 1 Stunde. Nach Erreichen des EndpH-Wertes von 8,5 wird die fertige Fällsuspension noch 2 Stunden bei 90°C gerührt und anschließend wie im Beispiel 1 und 2 weiterverarbeitet. Die Calcinierung des getrockneten Materials wurde bei Temperaturen von 430 bis 450°C durchgeführt. Die Reduktion wurde unter den gleichen Bedingungen wie im Beispiel 1 vorgenommen.

Die röntgenographische Untersuchung des getrockneten Zwischenproduktes ergab ein Gemisch aus Nickelhydroxynitrat (Ni₃(OH)₄(NO₃)₂), Nickelhydroxycarbonat (Ni₂(OH)₂CO₃ 4H₂O) und Nickelhydroxysilikat (Ni₃Si₂O₅(OH)₄).

Der fertige Katalysator enthält ca. 60 % Nickel, 16 % ZrO₂ und 5 % SiO₂ bezogen auf den Gesamtkatalysator. Die Maxima der Kristallitgrößen lagen bei 44 Angström und 101 Angström. Der Anteil an feindispersen Nickel, das heißt mit Kristallitgrößen von 30 bis 80 Angström, beträgt ca. 62 %.

Der Katalysator weist nach einer einstündigen Nachreduktion im Wasserstoffstrom bei 100°C einen Reduktionsgrad von 81 % auf.

Die Ergebnisse der katalytischen Testung sind in der Tabelle dargestellt.

### Beispiel 4 (Vergleichsbeispiel)

305,76 g Ni(NO₃)₂ 6 H₂O und 29,52 g Al(NO₃)₃ 9 H₂O werden in 1760 ml destilliertem Wasser und 2,32 g Zirconylcarbonat in 9 ml HNO₃ (56 Masse-%) gelöst. Beide Lösungen werden vereinigt und auf 101°C erhitzt. Diese Mischsalzlösung wird innerhalb von 3 Minuten gleichmäßig zu einer 100°C heißen und intensiv gerührten Sodalösung gegeben, die aus 147,04 g Na₂CO₃ und 1416 ml destilliertem Wasser hergestellt worden ist. In die frisch gefällte Suspension werden 27,76 g Kieselgur eingerührt und die dabei entstehende Mischung wird noch weitere 3 Minuten gerührt.

Das Fällprodukt wird anschließend filtriert und mit 70°C heißem Wasser gewaschen, bis der Alkaligehalt des Waschwassers ca. 20 mg Na₂O/l beträgt.

Der auf diese Weise erhaltene Filterkuchen wird in 70°C heißem Wasser suspendiert (Mengenverhältnis Filterkuchen zu Wasser = 1:1), 60 Minuten gerührt und danach erneut filtriert. Der danach anfallende Filterkuchen wird erneut filtriert. Der hierbei anfallende Filterkuchen wird zu zylinderförmigen Formkörpern (Durchmesser 5 mm, Länge 8 bis 10 mm) extrudiert und anschließend bei steigender Temperatur (50 bis 60°C) mit Luft auf einen Restgehalt an Wasser <10 Masse-%, bezogen auf getrocknete Masse, getrocknet. Das getrocknete Material wird im Wasserstoffstrom mit einer Belastung von 400 v/v h bei 470°C über einen Zeitraum von 4 Stunden reduziert.

Im getrockneten Zwischenprodukt wurden die Phasen Takovit (Carbonatvariante) und Nickelhydroxysilikat mit Carbonateinbau nachgewiesen.

Die Stabilisierung wurde wie in den erfindungsgemäßen Beispielen vorgenommen. Die Eigenschaften des Katalysators sind in der Tabelle denen der erfindungsgemäßen Katalysatoren gegenübergestellt.

### Beispiel 5

Die katalytische Charakterisierung der Katalysatoren wurde unter folgenden Bedingungen vorgenommen:

Hydrierung von Nitrobenzol zu Anilin im 0,5 l Rührautoklav mit Wasserstoffverbrauchsmessung von konstantem Druck:

| | |
|---|---|
| Katalysatormenge | 0,25 g |
| Reaktionsgemisch | 80 g Nitrobenzol und 40 ml H₂O |
| Reaktionsdruck | 30 bar |
| Reaktionstemperatur | 130°C |
| Rührgeschwindigkeit | 2000 Umdrehungen/Minute |

Als Maß für die Hydrieraktivität diente die Zeit, in der 100 % des Nitrobenzols umgesetzt worden sind. Die Stabilität der Katalysatoren wurde durch die Zunahme der durchschnittlichen Ni-Kristallitgröße nach einer einhundertstündigen Behandlung des Katalysators unter den Bedingungen des katalytischen Tests nach Abschluß der Umwandlung des Nitrobenzols zu Anilin charakterisiert.

Die XRD-Weitwinkel-Untersuchungen zur qualitativen Phasenzuordnung wurden unter den folgenden experimentellen Aufnahmebedingungen an einem Meßplatz der Fa. Rich. Seifert & Co. Freiberger Präzisionsmechanik GmbH ausgeführt:

| | |
|---|---|
| Generatordaten | 34kV/30mA |
| Goniometer | HZG4 |
| Strahlung | Cu-K_{á} |
| Filter | gebogener Graphitmonochromator |
| Winkelbereich | 2Θ = 10° - 70° |
| Schrittweite | ΔΘ = 0,05° |
| Zählzeit | 4 s |

Die Bearbeitung der Daten wurde im Auswertefile APX63 (SEIFERT FPM) durchgeführt. Zur Zuordnung der kristallinen Strukturen wurde der JCPDS-Auswertefile 1997 herangezogen.

Ebenfalls mit einem Meßplatz der Fa. Rich. Seifert & Co. Freiberger Präzisionsmechanik GmbH wurde die mittlere Primärteilchengröße des Nickels bestimmt, wobei die Streukurvenausschnitte senkrecht zur (111)-Netzebene aus der Interferenzlinienverbreiterung unter folgenden Bedingungen aufgenommen worden sind:

| | |
|---|---|
| Generatordaten | 40kV/30mA |
| Goniometer | XRD7 |
| Strahlung | Cu-K_{á} |
| Filter | Ni |
| Winkelbereich | 2Θ = 41°-49° |
| Schrittweite | ΔΘ = 0,05° |
| Zählzeit | 20 s |

Durch Anwendung des Peakentflechtungsprogramms PF4 der Firma Jandel Corporation wurden Aussagen zur Modalität (Monogauslinienprofil oder bimodales Gauslinienprofil) des Ni-(111)-Linienprofils gewonnen.

Die Meßdaten in der Tabelle belegen die Vorzüge der erfindungsgemäßen Katalysatoren: eine hohe katalytische Aktivität und sehr gute Stabilsierungseigenschaften, wie die geringen Zunahmen der Ni-Kristallitgröße zeigen.

**Tabelle**

| Katalysator | Reaktionszeit für 100-%-igen Umsatz | Ausbeute an Anilin in % | mittlere Ni-Kristallitgröße in Angström (vor der Reaktion) | mittlere Ni-Kristallitgröße in Angström (nach dem Stabilitätstest) |
|---|---|---|---|---|
| Beispiel 1 erfindungsgemäß | 86 | 99,9 | 73 | 83 |
| Beispiel 2 erfindungsgemäß | 88 | 99,85 | 78 | 91 |
| Beispiel 3 erfindungsgemäß | 84 | 99,9 | 66 | 79 |
| Beispiel 4 Vergleichsbeispiel | 121 | 99,8 | 107 | 138 |

## Patentansprüche

1. Katalysator, insbesondere für die Hydrierung von Nitrogruppen in Nitroaromaten, enthaltend Nickel auf einem Zirconium-haltigen Träger, wobei der Katalysator stabilisiert ist, Nickelkristallite mit bimodaler Nickelkristallitgrößenverteilung, einen Nickelgehalt von 60 bis 80 Masse-% (bezogen auf die Gesamtmasse des Katalysators), einen ZrO₂-Gehalt von 20 bis 40 Masse-% (bezogen auf die Gesamtmasse des Katalysators) sowie einen Reduktionsgrad (nach einer einstündigen Reduktion bei 100°C) von mindestens 70% aufweist.

2. Katalysator nach Anspruch 1, wobei die beiden Maxima der Nickelkristallitgrößenverteilung bei 30 bis 80 Angström und 81 bis 150 Angström liegen.

3. Katalysator nach einem der vorhergehenden Ansprüche, wobei der Anteil des Nickel mit Kristallitgrößen von 30 bis 80 Angström größer oder gleich 40 Masse-% (bezogen auf die Gesamtmasse des Katalysators) ist.

4. Katalysator nach einem der vorhergehenden Ansprüche, wobei der Träger ZrO₂, ZrO₂HfO₂, SiO₂ · ZrO₂, SiO₂ · ZrO₂HfO₂ oder Gemische zweier oder mehrerer dieser Substanzen enthält oder aus diesen besteht.

5. Katalysator nach Anspruch 4, wobei der SiO₂-Gehalt maximal 20 Masse-% (bezogen auf die Gesamtmasse des Katalysators) beträgt.

6. Katalysator nach Anspruch 4 oder 5, wobei der HfO₂-Gehalt maximal 4 Masse-% (bezogen auf die Gesamtmasse des Katalysators) beträgt.

7. Katalysator nach einem der vorhergehenden Ansprüche, wobei der Katalysator in Form eines Pulvers, insbesondere mit Korngrößen von 1 bis 100 µm, vorzugsweise 2 bis 25 µm vorliegt.

8. Verfahren zur Herstellung eines Nickel-haltigen Trägerkatalysators nach einem der Ansprüche 1 bis 7, insbesondere für die Hydrierung von Nitrogruppen in Nitroaromaten, enthaltend Nickel auf einem Zirconium-haltigen Träger, wobei der Katalysator stabilisiert ist, Nickelkristallite mit bimodaler Nickelkristallitgrößenverteilung, einen Nickelgehalt von 60 bis 80 Masse-% (bezogen auf die Gesamtmasse des Katalysators), einen ZrO₂-Gehalt von 20 bis 40 Masse-% (bezogen auf die Gesamtmasse des Katalysators) sowie einen Reduktionsgrad (nach einer einstündigen Reduktion) von mindestens 70 % aufweist, wobei durch Fällen aus einer Ni²⁺- und Zr⁴⁺-haltigen Lösung mit einer basischen Lösung bis zu einem pH-Endwert von 8 bis 9 ein Fällprodukt erhalten wird, welches bei Temperaturen von 250°C bis 650°C calciniert, mit Wasserstoff bei Temperaturen von 250°C bis 550°C reduziert und stabilisiert wird.

9. Verfahren nach Anspruch 8, wobei das Fällprodukt nach dem Calcinieren und vor dem Reduzieren inertisiert wird.

10. Verfahren nach Anspruch 8 oder 9, wobei die basische Lösung eine Lösung von NaOH, NaHCO₃, Na₂CO₃ oder eines Gemisches zweier oder mehrerer dieser Substanzen ist.

11. Verfahren nach einem der Ansprüche 8 bis 10, wobei die Ni²⁺- und Zr⁴⁺-haltige Lösung NO₃⁻ (Nitrat) und/oder Hf4⁺ enthält.

12. Verfahren nach einem der Ansprüche 8 bis 11, wobei die Ni²⁺- und Zr⁴⁺-haltige Lösung SiO₂, vorzugsweise in suspendierter Form, enthält.

13. Verfahren nach einem der Ansprüche 8 bis 12, wobei die Fällung bei Temperaturen von 50°C bis 95°C erfolgt.

14. Verfahren nach einem der Ansprüche 8 bis 13, wobei das Fällprodukt nach der Fällung filtriert, gewaschen, vorzugsweise mit Wasser, nachfolgend in nicht-reduzierender Atmosphäre getrocknet, vorzugsweise bei Temperaturen von 110°C bis 150°C, und ein Vorläuferkatalysator erhalten wird.

15. Verfahren nach einem der Ansprüche 8 bis 14, wobei bei der Herstellung des Vorläuferkatalysators Phasen aus Nickelhydroxynitrat (Ni₃(OH)₄(NO₃)₂) oder Nickelhydroxynitrat- (Ni₃(OH)₄(NO₃)₂) haltige Phasen, insbesondere Gemische aus Nickelhydroxynitrat (Ni₃(OH)₄(NO₃)₂), Nickelhydroxycarbonat (Ni₂(OH)₂CO₃ 4H₂O) und Nickelhydroxysilikat (Ni₃Si₂O₅(OH)₄) oder Gemische aus Nickelhydroxynitrat (Ni₃(CH)₄(NO₃)₂) und Nickelhydrogencarbonat (Ni(HCO₃)₂) oder Gemische aus Nickelhydroxynitrat (Ni₃(OH)₄(NO₃)₂) und Nickelhydroxid (Ni(OH)₂) mit Gitteraufweitungen ausgebildet werden.

16. Verfahren nach einem der Ansprüche 8 bis 15, wobei der Katalysatorvorläufer vor der Calcination zu Tabletten, Strängen, Kissen oder Kugeln geformt wird.

17. Verfahren nach einem der Ansprüche 8 bis 16, wobei der Katalysatorvorläufer nach der Calcination zu Tabletten, Strängen, Kissen oder Kugeln geformt wird.

18. Verfahren nach einem der Ansprüche 8 bis 17, wobei die Gasbelastung bei der Reduktion 500 bis 3000 v/v h beträgt.

19. Verfahren nach einem der Ansprüche 8 bis 18, wobei die Katalysatoren mit einem O₂-N₂-CO₂-Gemisch stabilisiert werden, insbesondere mit einem O₂-N₂-CO₂-Gemisch mit einem Sauerstoffgehalt von 0,1 bis 1 Vol.-% und einem CO₂-Gehalt von 0,6 Vol.-%.

20. Verfahren nach einem der Ansprüche 8 bis 19, wobei die Stabilisierung bei Temperaturen unterhalb 80°C stattfindet.

## Claims

1. Catalyst, especially for hydrogenating nitro groups in nitro aromatics, containing nickel on a zirconium-containing support, wherein the catalyst is stabilised and provides nickel crystallites with bimodal nickel-crystallite size distribution, a nickel content of 60 to 80% by mass (relative to the total mass of the catalyst), a ZrO₂ content from 20 to 40% by mass (relative to the total mass of the catalyst) as well as a degree of reduction (after one-hour reduction at 100°C) of at least 70%.

2. Catalyst according to claim 1, wherein the two maxima of the nickel-crystallite size distribution are at 30 to 80 Ångström and 81 to 150 Ångström.

3. Catalyst according to any one of the preceding claims, wherein the proportion of nickel with crystallite sizes from 30 to 80 Ångström is greater than or equal to 40% by mass (relative to the total mass of the catalyst).

4. Catalyst according to any one of the preceding claims, wherein the support contains or consists of ZrO₂, ZrO₂HfO₂, SiO₂ · ZrO₂, SiO₂ · ZrO₂HfO₂ or mixtures of two or more of these substances.

5. Catalyst according to claim 4, wherein the maximum SiO₂-content is 20% by mass (relative to the total mass of the catalyst).

6. Catalyst according to claim 4 or 5, wherein the maximum HfO₂-content is 4% by mass (relative to the total mass of the catalyst).

7. Catalyst according to any one of the preceding claims, wherein the catalyst is present in the form of a powder, especially with grain sizes from 1 to 100µm, preferably 2 to 25µm.

8. Method for producing a nickel-containing, supported catalyst according to any one of claims 1 to 7, especially for hydrogenating nitro groups in nitro aromatics, containing nickel on a zirconium-containing support, wherein the catalyst is stabilised and provides nickel crystallites with bimodal nickel-crystallite size distribution, a nickel content of 60 to 80% by mass (relative to the total mass of the catalyst), a ZrO₂-content of 20 to 40% by mass (relative to the total mass of the catalyst) and a degree of reduction (after a one-hour reduction) of at least 70%, wherein a precipitation product is obtained by precipitation from a solution containing Ni²⁺ and Zr⁴⁺ with a basic solution up to a final pH-value from 8 to 9, which calcines at temperatures from 250°C to 650°C and is reduced and stabilised with hydrogen at temperatures from 250°C to 550°C.

9. Method according to claim 8, wherein the precipitation product is rendered inert after calcination and before reduction.

10. Method according to claim 8 or 9, wherein the basic solution is a solution of NaOH, NaHCO₃, Na₂CO₃ or a mixture of two or more of these substances.

11. Method according to any one of claims 8 to 10, wherein the Ni²⁺- and Zr⁴⁺-containing solution contains NO₃⁻ (nitrate) and/or Hf⁴⁺.

12. Method according to any one of claims 8 to 11, wherein the Ni²⁺- and Zr⁴⁺-containing solution contains SiO₂, preferably in suspended form.

13. Method according to any one of claims 8 to 12, wherein the precipitation takes place at temperatures from 50°C to 95°C.

14. Method according to any one of claims 8 to 13, wherein the precipitation product is filtered, washed, preferably with water, after precipitation and subsequently dried in a non-reducing atmosphere, preferably at temperatures from 110°C to 150°C, and a precursor catalyst is obtained.

15. Method according to any one of claims 8 to 14, wherein, during the manufacture of the precursor catalyst, phases consisting of nickel hydroxy nitrate (Ni₃(OH)₄(NO₃)₂) or phases containing nickel hydroxy nitrate (Ni₃(OH)₄(NO₃)₂), especially mixtures of nickel hydroxy nitrate (Ni₃(OH)₄(NO₃)₂), nickel hydroxy carbonate (Ni₂(OH)₂CO₃4H₂O) and nickel hydroxy silicate (Ni₃Si₂O₅(OH)₄) or mixtures of nickel hydroxy nitrate (Ni₃(OH)₄(NO₃)₂) and nickel hydrogen carbonate (Ni(HCO₃)₂) or mixtures of nickel hydroxy nitrate (Ni₃(OH)₄(NO₃)₂) and nickel hydroxide (Ni(OH)₂) are formed with lattice expansions.

16. Method according to any one of claims 8 to 15, wherein the catalyst precursor is formed into tablets, strands, lumps or balls before calcination.

17. Method according to any one of claims 8 to 16, wherein the catalyst precursor is formed into tablets, strands, lumps or balls after calcination.

18. Method according to any one of claims 8 to 17, wherein the gas loading during reduction amounts to 500 to 3000 v/v h.

19. Method according to any one of claims 8 to 18, wherein the catalysts are stabilised with an O₂-N₂-CO₂-mixture, especially with an O₂-N₂-CO₂-mixture with an oxygen content from 0.1 to 1% by volume and a CO₂ content of 0.6% by volume.

20. Method according to any one of claims 8 to 19, wherein the stabilisation takes place at temperatures below 80°C.

## Revendications

1. Catalyseur, en particulier pour l'hydrogénation de groupes nitro dans des composés nitroaromatiques, contenant du nickel sur un support contenant du zirconium, dans lequel le catalyseur est stabilisé, et présente des cristallites de nickel ayant une distribution bimodale de la taille des cristallites de nickel, une teneur en nickel de 60 à 80 % en masse (par rapport à la masse totale du catalyseur), une teneur en ZrO₂ de 20 à 40 % en masse (par rapport à la masse totale du catalyseur) ainsi qu'un degré de réduction (après une heure de réduction à 100°C) d'au moins 70 %.

2. Catalyseur selon la revendication 1, **caractérisé en ce que** les deux maxima de la distribution de la taille des cristallites de nickel se situent de 30 à 80 Angström et de 81 à 150 Angström.

3. Catalyseur selon l'une quelconque des revendications précédentes, dans lequel la fraction de nickel d'une grandeur de cristallites de 30 à 80 Angström est supérieure ou égale à 40 % en masse (par rapport à la masse totale du catalyseur).

4. Catalyseur selon l'une quelconque des revendications précédentes, dans lequel le support contient ou est constitué de ZrO₂, ZrO₂HfO₂, SiO₂ . ZrO₂, SiO₂ . ZrO₂HfO₂ ou de mélanges de deux ou plusieurs de ces substances.

5. Catalyseur selon la revendication 4, dans lequel la teneur en SiO₂ est au maximum de 20 % en masse (par rapport à la masse totale du catalyseur).

6. Catalyseur selon la revendication 4 ou 5, dans lequel la teneur en HfO₂ est au maximum de 4 % en masse (par rapport à la masse totale du catalyseur).

7. Catalyseur selon l'une quelconque des revendications précédentes, dans lequel le catalyseur est présent sous forme d'une poudre, en particulier d'une granulométrie de 1 à 100 µm, de préférence de 2 à 25 µm.

8. Procédé de préparation d'un catalyseur sur support contenant du nickel selon l'une quelconque des revendications 1 à 7, en particulier pour l'hydrogénation de groupes nitro dans des composés nitroaromatiques, contenant du nickel sur un support contenant du zirconium, dans lequel le catalyseur est stabilisé, et présente des cristallites de nickel à distribution bimodale de la taille des cristallites de nickel, une teneur en nickel de 60 à 80 % en masse (par rapport à la masse totale du catalyseur), une teneur en ZrO₂ de 20 à 40 % en masse (par rapport à la masse totale du catalyseur), et un degré de réduction (après une heure de réduction) d'au moins 70 %, dans lequel on obtient, par précipitation à partir d'une solution contenant Ni²⁺ et Zr⁴⁺ au moyen d'une solution basique jusqu'à une valeur finale de pH de 8 à 9, un produit de précipitation, lequel est calciné à des températures de 250°C à 650°C, réduit au moyen de l'hydrogène à des températures de 250°C à 550°C et stabilisé.

9. Procédé selon la revendication 8, dans lequel le produit de précipitation est inertisé après la calcination et avant la réduction.

10. Procédé selon la revendication 8 ou 9, dans lequel la solution basique est une solution de NaOH, NaHCO₃, Na₂CO₃ ou un mélange de deux ou plusieurs de ces substances.

11. Procédé selon l'une quelconque des revendications 8 à 10, dans lequel la solution contenant Ni²⁺ et Zr⁴⁺ contient NO₃⁻ (nitrate) et/ou Hf⁴⁺.

12. Procédé selon l'une quelconque des revendications 8 à 11, dans lequel la solution contenant Ni²⁺ et Zr⁴⁺ contient SiO₂, de préférence sous une forme en suspension.

13. Procédé selon l'une quelconque des revendications 8 à 12, dans lequel la précipitation s'effectue à des températures de 50°C à 95°C.

14. Procédé selon l'une quelconque des revendications 8 à 13, dans lequel le produit de précipitation est, après la précipitation, filtré, lavé de préférence à l'eau, séché ensuite dans une atmosphère non réductrice, de préférence à des températures de 110°C à 150°C et un précurseur de catalyseur est obtenu.

15. Procédé selon l'une quelconque des revendications 8 à 14, dans lequel, lors de la préparation du précurseur de catalyseur, il se forme des phases constituées d'hydroxynitrate de nickel (Ni₃(OH)₄(NO₃)₂) ou des phases contenant de l'hydroxynitrate de nickel (Ni₃(OH)₄(NO₃)₂), en particulier des mélanges d'hydroxynitrate de nickel (Ni₃(OH)₄(NO₃)₂), d'hydroxycarbonate de nickel (Ni₂(OH)₂CO₃4H₂O) et d'hydroxysilicate de nickel (Ni₃Si₂O₅(OH)₄) ou des mélanges d'hydroxynitrate de nickel (Ni₃(OH)₄(NO₃)₂) et d'hydrogénocarbonate de nickel (Ni(HCO₃)₂) ou des mélanges d'hydroxynitrate de nickel (Ni₃(OH)₄(NO₃)₂) et d'hydroxyde de nickel (Ni(OH)₂) avec élargissement du réseau.

16. Procédé selon l'une quelconque des revendications 8 à 15, dans lequel le précurseur de catalyseur est, avant la calcination, façonné sous forme de pastilles, granulés cylindriques, coussinets ou billes.

17. Procédé selon l'une quelconque des revendications 8 à 16, dans lequel le précurseur de catalyseur est, après la calcination, façonné sous forme de pastilles, granulés cylindriques, coussinets ou billes.

18. Procédé selon l'une quelconque des revendications 8 à 17, dans lequel la charge de gaz lors de la réduction est de 500 à 3000 v/v h.

19. Procédé selon l'une quelconque des revendications 8 à 18, dans lequel les catalyseurs sont stabilisés au moyen d'un mélange O₂-N₂-CO₂, en particulier au moyen d'un mélange O₂-N₂-CO₂ d'une teneur en oxygène de 0,1 à 1 % en volume et d'une teneur en CO₂ de 0,6 % en volume.

20. Procédé selon l'une quelconque des revendications 8 à 19, dans lequel la stabilisation a lieu à des températures inférieures à 80°C.
